# EUROPÄISCHE PATENTSCHRIFT

(11) **EP 1 684 831 B1**
(45) Veröffentlichungstag und Bekanntmachung des Hinweises auf die Patenterteilung: **13.01.2010**
(21) Anmeldenummer: 04789821.8
(22) Anmeldetag: 29.10.2004
(51) Int. Cl.: A61M 5/20

(54) **VORRICHTUNG FÜR DIE VERABREICHUNG EINES INJIZIERBAREN PRODUKTS**
DEVICE FOR THE ADMINISTRATION OF AN INJECTABLE PRODUCT
DISPOSITIF POUR ADMINISTRER UN PRODUIT A INJECTER

(30) Priorität: 05.11.2003 DE 10351597; 05.11.2003 DE 10351596
(43) Veröffentlichungstag der Anmeldung: 02.08.2006
(73) Patentinhaber: TecPharma Licensing AG, 3401 Burgdorf (CH)
(72) Erfinder: HOMMANN, Edgar, CH-3257 Grossaffoltern (CH); SCHERER, Benjamin, CH-8610 Uster (CH)
(74) Vertreter: Schwabe - Sandmair - Marx
(86) Internationale Anmeldenummer: PCT/CH2004/000653
(87) Internationale Veröffentlichungsnummer: WO 2005/044346

(56) Entgegenhaltungen:
- EP-A- 0 897 728
- WO-A-00/62839
- WO-A-03/008023
- US-A- 5 092 842

## Beschreibung

Die Erfindung betrifft eine Vorrichtung für die Verabreichung eines injizierbaren Produkts. Die Vorrichtung kann insbesondere ein Injektionsgerät sein und ist vorzugsweise ein Autoinjektor. Der Erfindung geht es um das Entlüften, auch bekannt als Priming, eines mit dem Produkt gefüllten Reservoirs der Vorrichtung.

Ferner betrifft die Erfindung einen Autoinjektor für die Verabreichung eines injizierbaren Produkts.

Bei Injektionen sollte vermieden werden, dass mit dem zu injizierenden Produkt Luft injiziert wird. Dies gilt insbesondere für intravenöse Injektionen. Um die Injektion von Luft zu verhindern, werden das Reservoir und die sich stromabwärts anschließenden, produktführenden Komponenten entlüftet. Bei Injektionsgeräten, bei denen die Förderung des Produkts mittels eines Kolbens und einer auf den Kolben wirkenden Kolbenstange erfolgt, wird das Priming mittels eines kurzen Priminghubs der Kolbenstange und des Kolbens ausgeführt. Die Kolbenstange bildet die Primingeinrichtung oder zumindest das Abtriebsglied einer Primingeinrichtung. Um für das Priming nicht unnötig viel Produkt auszuschütten, wird der Priminghub der Kolbenstange voreingestellt. Die hierfür ausgeführten Primingeinrichtungen sind im Allgemeinen mechanisch komplex und filigran.

Schwierig gestaltet sich das Priming bei halbautomatischen Injektionsgeräten, bei denen der Verwender die Kolbenstange für die Produktausschüttung nicht selbst betätigen, sondern den Ausschütthub lediglich auslösen muss, die Kolbenstange also mittels eingebauter Vortriebseinrichtung bewegt wird. Insbesondere gilt dies für Autoinjektoren, d. h. für vollautomatische Injektionsgeräte, mittels denen nach Platzierung an einem gewünschten Ort der Produktverabreichung die Injektion durch Auslösung des Injektors vollautomatisch abläuft. Falls der Autoinjektor eine Einstechkanüle als infundierendes Teil umfasst, wird durch die Auslösung auch ein Einstechhub der Einstechkanüle automatisch ausgelöst.

Autoinjektoren sind zum Beispiel aus folgenden Dokumenten bekannt:
Die US-PS 4,031,893 beschreibt einen Autoinjektor mit einem Produktbehältnis, in dem für die Ausschüttung des Produkts ein Kolben in eine Vortriebsrichtung bewegbar aufgenommen ist. Der Kolben wird von einer Kolbenstange in die Vortriebsrichtung gedrückt. Die Kolbenstange wird in die Vortriebsrichtung mit der Kraft einer Ausschüttfeder beaufschlagt und gegen die Kraft der Feder in einer Halteposition in einem lösbaren Halteeingriff blockiert. Wenn der Halteeingriff gelöst wird, drückt die Kolbenstange gegen den Kolben. Aufgrund der Haftreibung des Kolbens wird in einer ersten Phase der Injektion der Kolben zusammen mit dem Produktbehältnis in die Vortriebsrichtung bewegt und dadurch eine Einstechkanüle in das Gewebe eingestochen. Sobald die Haftreibung des Kolbens überwunden ist, drückt die Kolbenstange den Kolben in dem Behältnis in die Vortriebsrichtung, bis das Behältnis vollständig entleert ist. Um bei gleichbleibender Behältnisgröße auch unterschiedliche Produktmengen pro Kolbenstangenhub verabreichen zu können, wird ein Adapter verwendet, der bei nur teilweise gefülltem Behältnis die Kolbenstange mit dem Kolben verbindet. Bei vollständig gefülltem Behältnis ist die Kolbenstange unmittelbar mit dem Kolben verbunden.

Aus der DE 198 22 031 B1 ist ein weiterer Autoinjektor bekannt, der für die Separierung der Einstechbewegung der Einstechkanüle und der Ausschüttbewegung des Kolbens eine Ablaufsteuerung aufweist. Zum einen wirkt wieder eine Kolbenstange auf einen in einem Produktbehältnis in eine Vortriebsrichtung bewegbar aufgenommenen Kolben, und zum anderen wirkt ein von der Kolbenstange separater Vortriebskörper in die Vortriebsrichtung auf das Behältnis. Eine Antriebsfeder wirkt auf den Vortriebskörper, der in einer Halteposition wieder in einem lösbaren Halteeingriff blockiert ist. Nach Lösen des Halteeingriffs bewegt sich der Vortriebskörper unter der Kraft der Feder in die Vortriebsrichtung und schiebt dabei das Behältnis mit der daran befestigten Einstechkanüle in die Vortriebsrichtung, so dass die Einstechkanüle in das Gewebe eingestochen wird. Diese Einstechbewegung wird durch einen Anschlag begrenzt. Sobald der Anschlag erreicht ist, löst sich die Kopplung zwischen dem Vortriebskörper und dem Behältnis, so dass die Feder den Vortriebskörper ohne das Behältnis weiter in die Vortriebsrichtung bewegt. Im Zuge dieser Bewegung gelangt er in Antriebskontakt mit der Kolbenstange und drückt die Kolbenstange und damit den Kolben in die Vortriebsrichtung, so dass das Produkt ausgeschüttet wird. Pro Ausschütthub wird das Behältnis jeweils entleert.

Aus der WO 00/62839 ist ein Injektorpen mit den Merkmalen des Oberbegriffs des Anspruchs 1 bekannt zum automatischen Vermischen von Substanzen einer Zweikammerampulle und einer daran anschließenden Entlüftung der Ampulle, dem so genannten Priming, vor der Injektion des Medikaments mittels des Pen. Das automatische Mischen wird mittels eines Antriebs bewirkt, der zum Beispiel eine Feder sein kann. Dieser Antrieb wirkt auf eine Hülse und über die Hülse auf eine Kolbenstange, die die Injektorkolbenstange umgibt. Durch den Vortrieb der Kolbenstange werden sowohl die Medikamentkomponenten vermischt, als auch der Injektor anschließend automatisch entlüftet, um eventuelle Luftbläschen im Medikament durch die Nadel hindurch ausgestoßen werden.

Es ist eine Aufgabe der Erfindung, eine Vorrichtung für die Verabreichung eines injizierbaren Produkts mit einer einfachen Primingeinrichtung zu schaffen. Die Primingeinrichtung soll insbesondere auch für halbautomatische und vollautomatische Injektionsgeräte geeignet sein.

Es ist eine weitere Aufgabe der Erfindung, einen Autoinjektor zu schaffen, der eine Produktmenge abgibt, die an das jeweils individuelle Bedürfnis leicht anpassbar ist.

Eine Vorrichtung für die Verabreichung eines injizierbaren Produkts, wie die Erfindung sie betrifft, ist in Anspruch 1 definiert. Sie umfasst ein Gehäuse, ein Reservoir für das Produkt, einen Kolben, eine Kolbenstange und eine Primingeinrichtung. Das Gehäuse kann das Reservoir unmittelbar bilden. Bevorzugt bildet jedoch ein von dem Gehäuse aufgenommenes Behältnis das Reservoir. Der Kolben ist in dem Reservoir in eine Vortriebsrichtung axial bewegbar aufgenommen, um durch eine Bewegung in die Vortriebsrichtung Produkt aus dem Reservoir auszuschütten. Die Kolbenstange ist in die Vortriebsrichtung bewegbar gelagert, um einen Ausschütthub auszuführen, bei dem sie in die Vortriebsrichtung auf den Kolben wirkend den Kolben in die Vortriebsrichtung bewegt. Die Kolbenstange kann mit dem Kolben axial fest verbunden sein, beispielsweise mittels einer Schraub- oder Bajonettverbindung oder einer einfachen Rastverbindung. Vorzugsweise wirkt sie jedoch nur über Druckkontakt auf dem Kolben, und es besteht über den bei einem Ausschütthub wirkenden Druckkontakt hinaus keine weitere Verbindung mit dem Kolben. In solch einer Ausführung kann eine durch einen Ausschütthub ausschüttbare Produktdosis ohne Längenänderung des Ausschütthubs eingestellt werden. Die Primingeinrichtung dient dem Entlüften des Reservoirs und der sich stromabwärts anschließenden, das Produkt führenden Komponenten der Vorrichtung. Die Primingeinrichtung ist ebenfalls bewegbar gelagert. Sie kann einen Priminghub ausführen, bei dem sie in die Vortriebsrichtung auf den Kolben wirkt, um die in dem produktführenden System möglicherweise vorhandene Luft zu verdrängen.

Die Primingeinrichtung ist nach der Erfindung relativ zu der Kolbenstange in die Vortriebsrichtung bewegbar. Sie führt den Priminghub relativ zu der Kolbenstange aus. Im Vergleich zu Primingeinrichtungen, bei denen die Kolbenstange nicht nur den Ausschütthub, sondern auch den Priminghub ausführt, sind keine mechanisch komplizierten Einstellmechanismen erforderlich, um den Priminghub einzustellen. Die Primingeinrichtung der Erfindung bildet eine weitere Kolbenstange, die allerdings nicht den Ausschütthub ausführen muss. Die den Ausschütthub ausführende Kolbenstange hingegen muss keine Primingfunktion übernehmen und insbesondere nicht für den im Vergleich zum Ausschütthub im Allgemeinen deutlich kürzeren Priminghub des Kolbens einstellbar sein.

Besonders vorteilhaft ist die erfindungsgemäße Primingeinrichtung für vollautomatische Injektionsgeräte, d. h. Autoinjektoren, und auch für halbautomatische Injektionsgeräte. Bei vollautomatischen und auch bei halbautomatischen Injektionsgeräten wird die Kolbenstange von der Elastizitätskraft einer Ausschüttfeder, vorzugsweise einer mechanischen Ausschüttfeder, in die Vortriebsrichtung getrieben und ist vor einer Auslösung in einer Halteposition gegen die Kraft der Ausschüttfeder gehalten und somit in die Vortriebsrichtung gespannt.

Ein voll- oder halbautomatisches Injektionsgerät umfasst vorzugsweise eine in die Vortriebsrichtung weisende Einstechkanüle als infundierendes Teil. Grundsätzlich kann das Injektionsgerät jedoch auch ein Druckinjektor ohne Einstechkanüle sein. Bei einem Autoinjektor mit Einstechkanüle ist die Einstechkanüle in die Vortriebsrichtung bewegbar, um für das Einstechen einen Einstechhub ausführen zu können. Hierbei wird es bevorzugt, wenn die Einstechkanüle axial an einem das Reservoir bildenden, relativ zu dem Gehäuse in die Vortriebsrichtung bewegbaren Behältnis axial festgelegt ist. Für das Einstechen kann eine Vortriebsstruktur, die mittels einer Einstechfeder in die Vortriebsrichtung getrieben wird, bei ihrer eigenen Bewegung in die Vortriebsrichtung das Behältnis mitnehmen. In alternativen Ausführungen wird die Vortriebsbewegung des Behältnisses, d. h. der Einstechhub, über die Haftreibung des Kolbens bewirkt. Hierfür kann die Ausschüttfeder auch gleichzeitig eine Einstechfeder bilden, indem sie in einer ersten Phase des Kolbenstangenhubs über die Kolbenhaftreibung das Behältnis und anschließend oder teilweise auch gleichzeitig den Kolben im Behältnis in die Vortriebsrichtung treibt.

Die vor Ausführung des Ausschütthubs oder Einstech- und Ausschütthubs relativ zu der Kolbenstange in die Vortriebsrichtung bewegbare Primingeinrichtung ist für alle genannten Ausführungen von Injektionsgeräten vorteilhaft und bedarf aufgrund ihres unabhängig von der Kolbenstange ausführbaren Priminghubs vorteilhafterweise kleiner durch den Typ des Injektionsgeräts bedingten Anpassung.

Erfindungsgemäß durchragt die Primingeinrichtung die Kolbenstange in die Vortriebsrichtung. Die Kolbenstange ist vorzugsweise hülsenförmig und umgibt die vorzugsweise stabförmige Primingeinrichtung. Wird die Kolbenstange für die Ausführung des Ausschütthubs von einer Ausschüttfeder beaufschlagt, ist die Ausschüttfeder zumindest über einen größeren Teil ihrer axialen Länge vorteilhafterweise in einem Ringspalt zwischen der Primingeinrichtung und der Kolbenstange angeordnet.

Die Vorrichtung erlaubt vorteilhafterweise die Einstellung einer bei einem Ausschütthub ausschüttbaren Produktdosis und umfasst hierfür ein Dosierelement. Das Dosierelement ist bewegbar angeordnet und über einen Dosiereingriff mit der Kolbenstange gekoppelt. Die Kopplung ist vorzugsweise so gestaltet, dass eine Dosierbewegung des Dosierelements eine Veränderung eines axialen Abstands, den die Kolbenstange vor Ausführung eines Ausschütthubs zu dem Kolben aufweist, bewirkt. In alternativen Ausführungen bewirkt die Dosierbewegung des Dosierelements eine Veränderung der axialen Länge des Ausschütthubs, was zweckmäßigerweise durch Verstellung eines Ausschüttanschlags, der den Ausschütthub in die Vortriebsrichtung begrenzt, verwirklicht wird.

Das Dosierelement kann in einer Doppelfunktion insbesondere auch ein Betätigungselement für die Primingeinrichtung bilden. In solchen bevorzugten Ausführungen ist das Dosierelement vorteilhafterweise in die Vortriebsrichtung bewegbar an dem Gehäuse angeordnet. Ein in die Vortriebsrichtung bewegbares Dosier- und Betätigungselement kann mit der Primingeinrichtung insbesondere so verbunden sein, dass es bei seiner Bewegung in die Vortriebsrichtung die Primingeinrichtung mitnimmt, vorzugsweise in die Vortriebsrichtung drückt. Das Dosier- und Betätigungselement führt in diesem Fall ebenfalls den Priminghub aus.

In bevorzugten Ausführungen koppelt die Primingeinrichtung das Dosierelement mit der Kolbenstange, d. h. die Primingeinrichtung überträgt die Dosierbewegung auf die Kolbenstange. Die Primingeinrichtung kann hierfür mit dem Dosierelement insbesondere so verbunden sein, dass das Dosierelement bei seiner Dosierbewegung die Primingeinrichtung mitnimmt, d. h. mit der Primingeinrichtung in Bezug auf die Dosierbewegung steif verbunden ist. Die Kopplung zwischen der Primingeinrichtung und der Kolbenstange kann bezüglich der Dosierbewegung ebenfalls vorteilhafterweise steif sein. Besonders bevorzugt sind bezüglich der Dosierbewegung sowohl die Kopplung zwischen dem Dosierelement und der Primingeinrichtung als auch die Kopplung zwischen der Primingeinrichtung und der Kolbenstange steif.

Falls die Vorrichtung die Einstellung der Produktdosis erlaubt, umfasst sie vorteilhafterweise auch eine Dosisanzeige. Die Dosisanzeige umfasst ihrerseits wenigstens zwei Anzeigeelemente, von denen das eine eine Dosisskala und das andere einen Zeiger der Dosisanzeige bildet, dessen Position auf der Dosisskala die eingestellte Produktdosis anzeigt. Eines der Anzeigeelemente ist mit der Primingeinrichtung so gekoppelt, dass durch eine Bewegung der Primingeinrichtung in die Vortriebsrichtung die Position des Zeigers auf der Dosisskala verstellt wird. Die Verstellung ist vorteilhafterweise so, dass nach Ausführung eines Priminghubs, falls dieser vor Einstellung der Produktdosis ausgeführt wird, die noch ausschüttbare Dosis, vorzugsweise die bei dem nächsten Ausschütthub ausschüttbare Dosis, angezeigt wird.

Bevorzugten Ausführungen entspricht es, wenn die Primingeinrichtung die Dosierbewegung des Dosierelements auf die Kolbenstange überträgt und mit einem der Anzeigeelemente so gekoppelt ist, dass die durch die Dosierbewegung des Dosierelements bewirkt Bewegung der Primingeinrichtung eine Verstellung der Dosisanzeige zur Folge hat. Von der Ausschüttbewegung der Kolbenstange ist die Primingeinrichtung jedoch vorzugsweise entkoppelt. Grundsätzlich wäre es jedoch denkbar, dass die Primingeinrichtung die Ausschüttbewegung der Kolbenstange mitmacht, aber für die Ausführung des Priminghubs relativ zu der Kolbenstange bewegbar ist.

Nach einem weiteren Aspekt ist ein nicht beanspruchter Autoinjektor vorgesehen, der ein Gehäuse, ein von dem Gehäuse aufgenommenes Produktbehältnis, einen in dem Produktbehältnis in eine Vortriebsrichtung axial bewegbar aufgenommenen Kolben, eine Kolbenstange und eine Ausschüttfeder umfasst. Die Ausschüttfeder beaufschlagt die Kolbenstange mit einer in die Vortriebsrichtung wirkenden Kraft. Die Kolbenstange ist gegen die Kraft der Ausschüttfeder in einer Halteposition in einem lösbaren Halteeingriff blockiert. Die Blockierung besteht letztlich relativ zu dem Gehäuse und kann sogar ein Halteeingriff unmittelbar zwischen der Kolbenstange und dem Gehäuse sein. Vorzugsweise ist die Kolbenstange jedoch an einer ebenfalls axial bewegbaren Struktur blockiert, die ihrerseits in einer Halteposition in einem lösbaren Halteeingriff mit dem Gehäuse ist. Wird der Halteeingriff der Kolbenstange gelöst, so führt die Kolbenstange angetrieben durch die Kraft der Ausschüttfeder einen Ausschütthub aus, durch den der Kolben in dem Behältnis in die Vortriebsrichtung bewegt und eine vorgegebene Produktdosis ausgeschüttet wird.

Der Autoinjektor ist mit einem mit dem Gehäuse bewegbar verbundenen Dosierelement ausgestattet, und das Dosierelement ist mit der Kolbenstange gekoppelt. Das Dosierelement ist betätigbar und kann eine Dosierbewegung ausführen. Die Kopplung mit der Kolbenstange ist derart, dass durch die Dosierbewegung des Dosierelements eine durch den Ausschütthub der Kolbenstange ausschüttbare Produktdosis einstellbar ist. Es wird somit auch bei einem Autoinjektor die individuelle Einstellung der Produktdosis, die bei der Injektion verabreicht wird, nicht nur beim Hersteller möglich, sondern auch durch einen Arzt, klinisch geschultes Personal oder sogar durch die Person, der die Produktdosis verabreicht wird oder die sich die Produktdosis selbst verabreicht. Die Kopplung umfasst einen auf Formschluss beruhenden Dosiereingriff, in dem die Dosierbewegung eine axiale Verstellbewegung bewirkt.

Die Kolbenstange ist mit dem Kolben vorzugsweise nicht verbunden, sondern drückt bei der Produktausschüttung mit einem in Vortriebsrichtung vorderen Ende lediglich gegen die Kolbenrückseite. In dieser bevorzugten Ausführung, aber auch in Ausführungen, in denen die Kolbenstange mit dem Kolben verbunden ist, kann die Produktdosis beispielsweise dadurch eingestellt werden, dass ein den Ausschütthub in die Vortriebsrichtung begrenzender Anschlag durch die Dosierbewegung des Dosierelements axial verstellt wird. Bevorzugt wird es jedoch, wenn der Ausschütthub ungeachtet der eingestellten Produktdosis stets gleich lang ist, was bei einer nur lose gegen den Kolben drückenden Kolbenstange durch die Verstellung des axialen Abstands zwischen der Kolbenstange und dem Kolben bewirkt werden kann, wie dies bevorzugt wird.

In solchen Ausführungen kann beispielsweise die Halteposition der Kolbenstange axial verstellt werden.

Bevorzugten Ausführungen entspricht es jedoch, wenn die Kolbenstange selbst längenveränderbar ist und die Dosierbewegung des Dosierelements die Längenverstellung bewirkt. Für die Längenverstellbarkeit ist die Kolbenstange mehrteilig, wobei von den mehreren Kolbenstangenteilen wenigstens zwei miteinander in dem Dosiereingriff sind, in dem sie relativ zueinander axial verstellt werden. Ein erstes der Kolbenstangenteile kontaktiert für die Produktausschüttung den Kolben. Ein anderes der Kolbenstangenteile ist in der Halteposition der Kolbenstange in dem Halteeingriff. Eines der Kolbenstangenteile kann in dem Dosiereingriff gegen das andere vorzugsweise um eine in die Vortriebsrichtung weisende Achse verdreht werden, wobei der Dosiereingriff die Drehbewegung in eine relative Axialbewegung umwandelt. Nicht zuletzt wegen seiner Einfachheit, sondern auch wegen der Möglichkeit der kontinuierlichen Längenverstellung wird ein Gewindeeingriff als Dosiereingriff favorisiert.

Die mehreren Teile der Kolbenstange sind so miteinander verbunden, dass sie den Ausschütthub gemeinsam ausführen. Insbesondere kann der Dosiereingriff über die Längenverstellung hinaus auch bewirken, dass die den Dosiereingriff bildenden Kolbenstangenteile einander bei dem Ausschütthub mitnehmen und den Ausschütthub somit gemeinsam ausführen.

In einer ersten Ausführung besteht die Kolbenstange aus zwei derartigen Kolbenstangenteilen, die miteinander in dem Dosiereingriff sind. In einer zweiten Ausführung wird die Kolbenstange von mehr als zwei Kolbenstangenteilen gebildet, von denen zwei in dem Dosiereingriff miteinander sind. Gegebenenfalls können die mehreren Kolbenstangenteile je paarweise miteinander in je einem Dosiereingriff sein. In einer bevorzugten Ausführung mit drei Kolbenstangenteilen oder gegebenenfalls auch noch mehr Kolbenstangenteilen, ist ein erstes der Kolbenstangenteile in dem Dosiereingriff axial verstellbar, ein zweites der Kolbenstangenteile ist in dem Halteeingriff und ein drittes bildet die Kopplung oder einen Teil der Kopplung zwischen dem Dosierelement und dem axial verstellbaren ersten Kolbenstangenteil. Das dritte Kolbenstangenteil wird durch die Dosierbewegung vorzugsweise axial nicht verstellt.

Die Dosierbewegung des Dosierelements ist vorzugsweise eine Drehbewegung relativ zu dem Gehäuse. Besonders bevorzugt führt das Dosierelement die Drehbewegung um eine in die Vortriebsrichtung weisende Drehachse aus. Falls im Falle der bevorzugten Längenverstellbarkeit der Kolbenstange auch eines ihrer Kolbenstangenteile drehverstellbar relativ zu einem anderen Kolbenstangenteil ist, wird es bevorzugt, wenn die Drehachse des Dosierelements und die des drehverstellbaren Kolbenstangenteils fluchten. Das Dosierelement und das drehverstellbare Kolbenstangenteil können besonders einfach auf der gleichen Drehachse in der Halteposition der Kolbenstange verdrehsteif miteinander verbunden sein.

Vorzugsweise ist das erste Kolbenstangenteil mit dem Dosierelement gekoppelt, so dass es durch dessen Dosierbewegung relativ zu dem in dem Halteeingriff befindlichen zweiten Kolbenstangenteil verstellt wird. Obgleich weniger bevorzugt, wäre es jedoch durchaus möglich, das zweite Kolbenstangenteil durch Kopplung mit dem Dosierelement im Halteeingriff zu verstellen, indem im Dosiereingriff entweder die axiale Position des zweiten Kolbenstangenteils ohne Lösen des Halteeingriffs oder die axiale Position des ersten Kolbenstangenteils verstellt wird.

Das erste Kolbenstangenteil durchragt vorzugsweise das zweite, d. h. das zweite Kolbenstangenteil umgibt das erste zumindest über einen axialen Längenabschnitt. Die Ausschüttfeder ist vorzugsweise in einem zwischen den Kolbenstangenteilen verbleibenden Ringspalt aufgenommen. Sie ist in die Vortriebsrichtung vorzugsweise unmittelbar an dem zweiten Kolbenstangenteil abgestützt oder drückt das genannte dritte Kolbenstangenteil gegen das zweite.

Vorzugsweise wird bei dem Ausschütthub auch das in der Halteposition in dem Halteeingriff befindliche zweite Kolbenstangenteil in das Behältnis hinein bewegt. In der Einstellung, in der die Kolbenstange ihre kleinste Länge aufweist, sind die Kolbenstangenteile axial vorzugsweise auf Anschlag.

Der Autoinjektor ist in bevorzugten Ausführungen mit einer Dosisanzeige ausgestattet. Die Dosisanzeige umfasst zwei Anzeigeelemente, die eine Dosisskala und einen Zeiger bilden, dessen Position auf der Dosisskala die eingestellte Produktdosis anzeigt.

Bevorzugt bildet ein Hülsenkörper, der den Zeiger umgibt, die Dosisskala und ist zum Ablesen der Position des Zeigers zumindest im Bereich der Dosisskala durchsichtig. Das die Dosisskala bildende Anzeigeelement kann insbesondere ein Dosierknopf sein, der an einem proximalen Ende des Gehäuses angeordnet und das Dosierelement der Dosiseinstellung ist.

Der Zeiger der Dosisanzeige kann im Falle der Realisierung der Dosiseinstellung mittels einer längenverstellbaren Kolbenstange insbesondere unmittelbar mit demjenigen Kolbenstangenteil verbunden sein, das für die Dosiseinstellung axial verstellt wird. Die Verbindung mit dem betreffenden Kolbenstangenteil ist so gestaltet, dass der Zeiger die axiale Dosiseinstellbewegung des betreffenden Kolbenstangenteils mitmacht. Bei dem Ausschütthub sollte die Verbindung sich selbsttätig lösen und der Zeiger im Bereich der Dosisskala verbleiben. In einer anderen, besonders bevorzugten Ausführung ist der Zeiger von der axialen Verstellbewegung des axial verstellbaren Kolbenstangenteils oder eines gegebenenfalls axial verstellbaren Kolbenstangenanschlags entkoppelt, aber an die Dosierbewegung, die die axiale Verstellbewegung bewirkt, gekoppelt. Die Kopplung umfasst ein Getriebe, vorzugsweise ein Untersetzungsgetriebe, das die axiale Einstellbewegung der Kolbenstange oder eines Kolbenstangenanschlags in eine Axialbewegung des Zeigers übersetzt oder bevorzugter untersetzt, vorzugsweise proportional. Ist der Dosiereingriff ein Gewindeeingriff, so kann das Untersetzungsgetriebe insbesondere ebenfalls ein Gewindeeingriff sein, mit einem Gewinde, das eine andere, vorzugsweise geringere Steigung als das Gewinde des Dosiereingriffs aufweist.

Eines der beiden genannten Anzeigeelemente ist vorteilhafterweise in der axialen Flucht der Kolbenstange angeordnet und kann sogar, wie geschildert, unmittelbar auf der Kolbenstange sitzen. Bevorzugt sind beide Anzeigeelemente in der Flucht der Kolbenstange angeordnet. Insbesondere kann ein die Dosierbewegung des Dosierelements auf die Kolbenstange übertragender Stab in dem Gewindeeingriff mit dem betreffenden Anzeigeelement, vorzugsweise dem Zeiger, sein.

Bevorzugte Merkmale werden auch in den Unteransprüchen und deren Kombinationen beschrieben. Die dadurch offenbarten und die vorstehend geschilderten Merkmale ergänzen einander wechselseitig.

Nachfolgend werden Ausführungsbeispiele der Erfindung anhand von Figuren erläutert. An den Ausführungsbeispielen offenbar werdende Merkmale bilden je einzeln und in jeder Merkmalskombination die Gegenstände der Ansprüche und auch die vorstehend geschilderten Ausgestaltungen vorteilhaft weiter. Es zeigen:
- Figur 1: ein erstes nicht beanspruchtes Ausführungsbeispiel eines dosisvariablen Autoinjektors in der Einstellung "Maximaldosis"
- Figur 2: den Autoinjektor des ersten Ausführungsbeispiels in der Einstellung "Minimaldosis",
- Figur 3: den Autoinjektor der Figur 2 mit vorgeschobener Einstechkanüle, aber vor einer Produkkausschüttung,
- Figur 4: den Autoinjektor der Figur 2 mit vorgeschobener Einstechkanüle und nach der Produktausschüttung,
- Figur 5: den Autoinjektor der Figur 1 in einer Vergrößerung,
- Figur 6: ein zweites nicht beanspruchtes Ausführungsbeispiel eines dosisvariablen Autoinjektors,
- Figur 7: ein erfindungsgemässes drittes Ausführungsbeispiel eines dosisvariablen Autoinjektors, der zusätzlich mit einer Primingeinrichtung ausgestattet ist,
- Figur 8: ein erfindungsgemässes viertes Ausfuhrungsbeispiel eines dosisvariablen Autoinjektors in der Einstellung "Minimaldosis" und mit einer Primingeinrichtung,
- Figur 9: den Autoinjektor des vierten Ausführungsbeispiels nach dem Primen,
- Figur 10: den Autoinjektor des vierten Ausführungsbeispiels in der Einstellung "Maximaldosis" und
- Figur 11: eine Vergrößerung aus Figur 10.

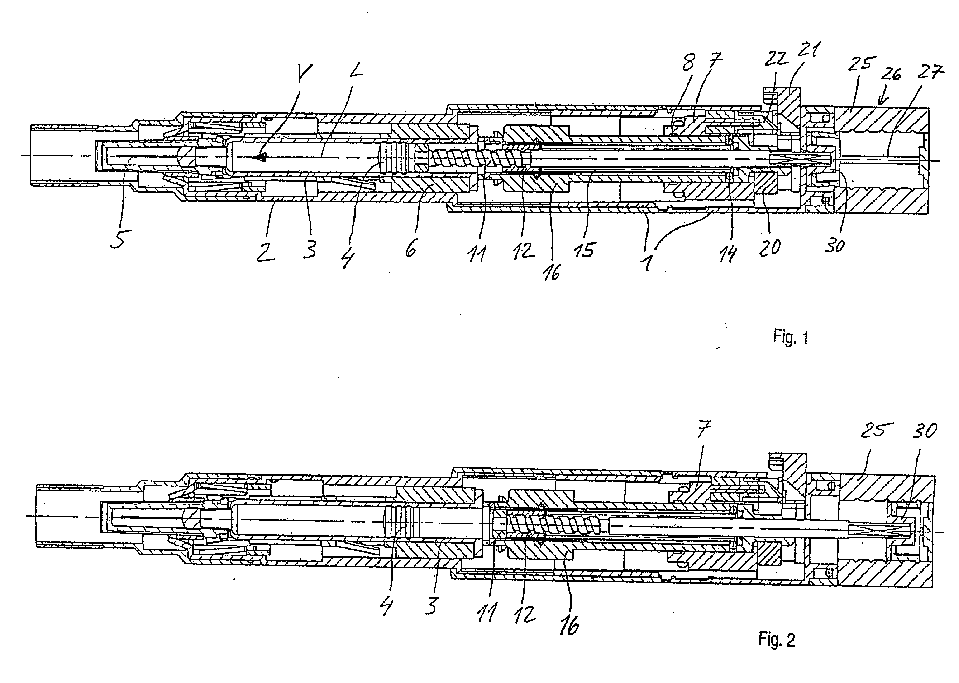

Figur 1 zeigt einen dosisvariablen Autoinjektor, d. h. einen Autoinjektor, an dem vom Verwender die für die Injektion gewünschte Dosis eines zu verabreichenden Produkts einstellbar ist. Bei dem Produkt handelt es sich vorzugsweise um eine Medikamentenflüssigkeit, beispielsweise Insulin, ein Wachstumshormon oder ein Osteoporosepräparat. Figur 1 zeigt den Injektor in einer Einstellung, die einer Maximaldosis entspricht, d. h. im Falle einer Auslösung wird die mit dem Injektor maximal verabreichbare Produktdosis ausgeschüttet.

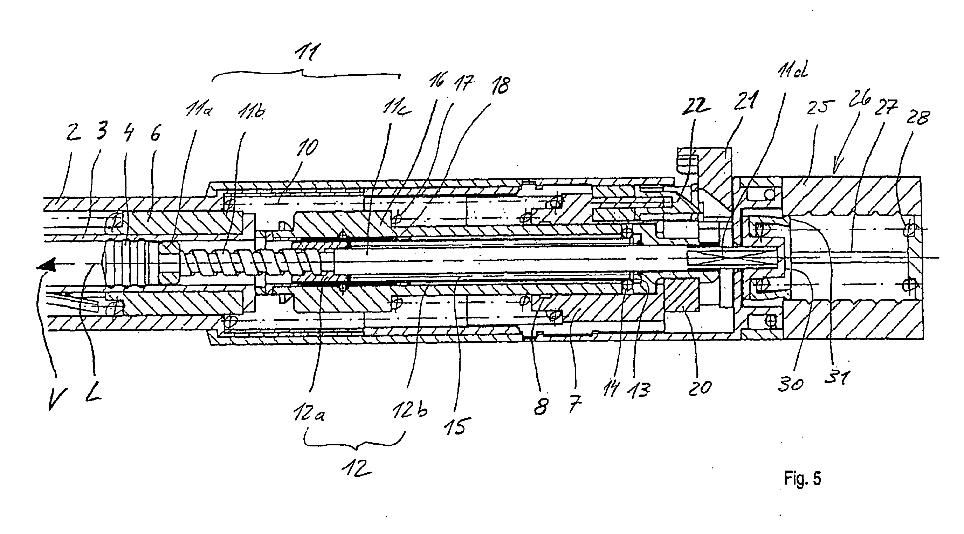

Figur 5 zeigt einen Teil des Injektors in einer Vergrößerung, in der Bezugszeichen auch für Komponenten des Injektors eingetragen sind, die in Figur 1 zur Wahrung der Übersichtlichkeit nicht mit Bezugszeichen versehen sind. Für die Beschreibung des ersten Ausführungsbeispiels sei daher stets auch auf Figur 5 verwiesen.

Der Injektor umfasst ein Gehäuse, das im Wesentlichen aus einer ersten Gehäusehülse 1 und einer zweiten Gehäusehülse 2 besteht, die entlang einer gemeinsamen, zentralen Längsachse L relativ zu der ersten Gehäusehülse 1 bewegbar ist. Die erste Gehäusehülse 1 bildet eine axiale Gleitführung für die zweite Gehäusehülse 2. In der Gehäusehülse 2 ist ein mit dem Produkt gefülltes Behältnis 3 entlang der Längsachse L in eine Vortriebsrichtung V bewegbar aufgenommen. An dem bezüglich der Vortriebsrichtung V vorderen, distalen Ende des Behältnisses 3 ist eine Einstechkanüle 5 entlang der Längsachse L in die Vortriebsrichtung V weisend befestigt. Ein Kolben 4 schließt das Behältnis 3 an seinem bezüglich der Vortriebsrichtung hinteren, proximalen Ende dicht ab. Der Kolben 4 ist in die Vortriebsrichtung V auf einen Auslass des Behältnisses zu bewegbar, um durch solch eine Ausschüttbewegung das Produkt aus dem Behältnis 3 auszustoßen und durch die Einstechkanüle 5 auszuschütten. Für die bewegliche Lagerung des Behältnisses 3 sorgt ein Behältnishalter 6, in den das Behältnis 3 in die Vortriebsrichtung V bis gegen einen Anschlag eingeschoben ist. Die Gehäusehülse 2 bildet eine axiale Linearführung für den Behältnishalter 6 und somit für das Behältnis 3. Die Gehäusehülse 2 umgibt nicht nur den Behältnishalter 6 mit dem Behältnis 3, sondern auch die Einstechkanüle bis über deren Spitze hinaus. Die Gehäusehülse 2 bildet einen Nadelschutz.

Eine Kolbenstange bewirkt den Vortrieb des Kolbens 4. Die Kolbenstange besteht aus zwei Kolbenstangenteilen 11 und 12, die miteinander in einem Dosiereingriff sind, in dem das Kolbenstangenteil 11 bei Ausführung einer Dosierbewegung eine axiale Verstellbewegung relativ zu dem Kolbenstangenteil 12 ausführt. Der Dosiereingriff ist formschlüssig.

Das erste Kolbenstangenteil 11 ist stabförmig und umfasst einen Stempel 11a, daran anschließend einen Eingriffsabschnitt in Form eines Gewindeabschnitts 11b und schließlich einen Schaft 11c mit einem Kopplungsabschnitt 11d am Ende. Das Kolbenstangenteil 11 wirkt allein durch Andrücken in die Vortriebsrichtung V auf den Kolben 4. Über den Druckkontakt hinaus besteht zwischen dem Kolbenstangenteil 11 und dem Kolben 4 keine Verbindung.

Das Kolbenstangenteil 11 durchragt das Kolbenstangenteil 12. Das Kolbenstangenteil 12 wird von einer Haltehülse 12b und einem Eingriffsteil 12a gebildet. Das Eingriffsteil 12a ist in die Hülse 12b an deren vorderen Ende eingepresst. Das Eingriffsteil 12a ist mit dem Kolbenstangenteil 11 in dem Dosiereingriff, im Ausführungsbeispiel in einem Gewindeeingriff mit dem Gewindeabschnitt 11b.

Die Kolbenstange 11, 12 ist in einer Vortriebsstruktur 16 in die Vortriebsrichtung V axial bewegbar gelagert und wird bei solch einer Bewegung, der Ausschüttbewegung, von der Vortriebsstruktur 16 linear geführt. Eine distale Linearführung wird von einer in die Vortriebsstruktur 16 eingepressten Hülse gebildet, die das Kolbenstangenteil 12 gleitend führt und einen Anschlag 18 bildet, der die Ausschüttbewegung der Kolbenstange 11, 12 in Vortriebsrichtung V begrenzt. Eine proximale Linearführung ist zwischen der Vortriebsstruktur 16 und dem Kolbenstangenteil 11 gebildet. In einem Ringspalt zwischen dem Kolbenstangenteil 11 und dem Kolbenstangenteil 12 ist eine Ausschüttfeder 15 aufgenommen, im Ausführungsbeispiel eine Schraubenfeder, die sich gegen die Vortriebsrichtung V an der Vortriebsstruktur 16 und in die Vortriebsrichtung V an dem Kolbenstangenteil 12, im Ausführungsbeispiel an dessen Eingriffsteil 12a, abstützt und die Kolbenstange 11, 12 mit einer Federkraft beaufschlagt. Die Kolbenstange 11, 12 wird gegen die Kraft der gespannten Ausschüttfeder 15 in einer proximalen Halteposition gehalten bzw. blockiert, indem das Kolbenstangenteil 12 mit der Vortriebsstruktur 16 in einem lösbaren Halteeingriff ist. Für den Halteeingriff weitet sich die Hülse 12b an ihrem proximalen Ende nach auswärts auf, wodurch eine zu der Längsachse L geneigte Halteschulter 13 erhalten wird. Die Halteschulter 13 hintergreift mehrere Blockierelemente 14, die in Durchbrüchen der Vortriebsstruktur 16 quer beweglich zu der Längsachse L geführt werden. Die Blockierelemente 14 sind Zylinderstifte, könnten aber beispielsweise auch durch Kugeln gebildet werden. Die Blockierelemente, 14 werden von der Halteschulter 13 nach radial auswärts mit einer Druckkraft beaufschlagt. In der Halteposition der Kolbenstange 11, 12 wird ein Ausweichen der Blockierelemente 14 durch einen Lagerblock 7 verhindert, der die Vortriebsstruktur 16 axial bewegbar führt und in dem die Blockierelemente 14 aufnehmenden Axialabschnitt umgibt. Der Lagerblock 7 ist fest mit dem Gehäuse 1 verbunden.

Eine Einstechfeder 17 spannt die Vortriebsstruktur 16 in die Vortriebsrichtung V gegen das Behältnis 3. Die Vortriebsstruktur 16 wird gegen die Kraft der Einstechfeder 17 in einem ebenfalls lösbaren Halteeingriff in einer proximalen Halteposition blockiert. In dem Halteeingriff hintergreift die Vortriebsstruktur 16 ein Blockierelement 20. Das Blockierelement 20 kann durch Betätigung eines Auslöseelements 21 über ein Übertragungsstück 22 quer zu der Längsachse L aus dem Halteeingriff mit der Vortriebsstruktur 16 bewegt werden. Das Auslöseelement 21 ist ein Auslöseknopf, der aus der Gehäusehülse 1 vorragt und für das Auslösen einfach einwärts gedrückt wird.

Das zweite Kolbenstangenteil 12 wird über die Vortriebsstruktur 16 und den Lagerblock 7 relativ zu der Gehäusehülse 1 verdrehsicher gelagert.

Die Antriebseinheit, bestehend im Wesentlichen aus der Vortriebsstruktur 16, der Kolbenstange 11, 12 und den beiden Federn 15 und 17, befindet sich im geladenen Zustand, in dem die Vortriebsstruktur 16 in ihrer proximalen Halteposition zur Gehäusehülse 1 und die Kolbenstange 11, 12 in ihrer proximalen Halteposition zur Vortriebsstruktur 16 blockiert sind. In diesem Zustand kann die Produktdosis eingestellt werden, die nach Auslösung der Antriebseinheit ausgeschüttet wird. Um diese Produktdosis einzustellen, wird die axiale Position des Kolbenstangenteils 11 relativ zu dem axial fixierten Kolbenstangenteil 12 eingestellt. Durch die Einstellung wird der axiale lichte Abstand zwischen dem Kolben und dem Stempel 11a verändert. Die axiale Länge des Ausschütthubs der Kolbenstange 11, 12 ist durch die proximale Halteposition der Kolbenstange 11, 12 und den Anschlag 18 vorgegeben und wird durch die Einstellung nicht beeinflusst. Vielmehr wird durch die Einstellung des axialen lichten Abstands innerhalb des Ausschütthubs die axiale Weglänge vorgegeben, über die das Kolbenstangenteil 11 gegen den Kolben 4 drückt und diesen dadurch vortreibt.

In Figur 1 nimmt das Kolbenstangenteil 11 relativ zu dem Kolbenstangenteil 12 seine distalste Position ein, in der es in der Halteposition der Kolbenstange 11, 12 zum Kolben 4 den minimalen Abstand aufweist, der "null" sein kann. In dieser Einstellung legen die Kolbenstange 11, 12 und der Kolben 4 bei einem Ausschütthub die größte gemeinsame Weglänge zurück, und es wird die größte einstellbare Produktdosis, d. h. die Maximaldosis, ausgeschüttet.

Figur 2 zeigt den Injektor ebenfalls im geladenen Zustand, allerdings in der Einstellung Minimaldosis". Das Kolbenstangenteil 11 nimmt relativ zu dem Kolbenstangenteil 12 seine proximalste Position ein, in der der Stempel 11a Anschlagkontakt mit der Stirnseite des Kolbenstangenteils 12a hat. Der axiale lichte Abstand zwischen dem Kolben 4 und der Kolbenstange 11, 12 ist in der Halteposition maximal und die Weglänge, über die die Kolbenstange 11, 12 im Verlaufe eines Ausschütthubs gegen den Kolben 4 drückt, ist minimal.

Der Injektor ist für den einmaligen Gebrauch bestimmt und wird nach der Injektion entsorgt. Er wird mit dem die Maximaldosis enthaltenden Behältnis 3 im geladenen Zustand an den Verwender abgegeben, beispielsweise eine Person, die sich das Produkt selbst verabreicht, oder beispielsweise an Ärzte. Der Verwender stellt die gewünschte Produktdosis durch die Einstellung der Länge der Kolbenstange 11, 12 selbst und in diesem Sinne individuell ein.

Die Einstellung wird mittels eines Dosierelements 25 vorgenommen, das relativ zu der Gehäusehülse 1 eine Dosierbewegung ausführen kann. Im Ausführungsbeispiel ist das Dosierelement 25 an der Gehäusehülse 1 um die Längsachse L drehbar befestigt, d. h. die Dosierbewegung ist eine Drehbewegung um die Längsachse L. Zur Übertragung der Dosierbewegung ist das Dosierelement 25 mittels eines Koppelements 27 mit der Kolbenstange 11, 12 um die Längsachse L verdrehgesichert gekoppelt. Allerdings kann das Kolbenstangenteil 11 relativ zu dem Koppelelement 27 axial bewegt werden. Das Koppelelement 27 ist mit dem Dosierelement 25 verdrehgesichert verbunden. Im Ausführungsbeispiel ist es einstückig mit dem Dosierelement 25 als Längsrippe des Dosierelements 25 geformt.

Das Dosierelement 25 ist in seinem Hülsenteil mit einer Dosisskala 26 versehen, in der Dosiseinheiten von proximal nach distal zunehmend aufgetragen sind. Mit seiner Dosisskala 26 bildet das Dosierelement 25 ein erstes Anzeigeelement einer Dosisanzeige. Ein zweites Anzeigeelement 30 der Dosisanzeige wird von einer Feder 28 auf das proximale Ende des Kolbenstangenteils 11 gedrückt und ist als hütchenförmiger Aufsatz geformt. Das zweite Anzeigeelement 30 bildet einen Zeiger 31, dessen axiale Position auf der Dosisskala 26 abgelesen werden kann. Das Dosierelement 25 ist hierfür im Bereich der Dosisskala 26 oder vorzugsweise im Ganzen so weit durchsichtig, dass der Zeiger 31 durch den Hülsenteil des Dosierelements 25 erkennbar ist.

Das Koppelelement 27 greift in eine Längsnut des Anzeigeelements 30 ein, wodurch die Anzeigeelemente 25 und 30 um die Längsachse verdrehgesichert, aber relativ zueinander axial bewegbar miteinander verbunden sind. Das Anzeigeelement 30 sitzt verdrehgesichert auf dem Kopplungsabschnitt 11d des Kolbenstangenteils 11. Im Ergebnis wird durch die Kopplung über 27 und 30 das Dosierelement 25 drehsteif mit dem Kolbenstangenteil 11 verbunden.

Der Ablauf einer Produktverabreichung ist aus der Sequenz der Figuren 1 bis 4 ersichtlich.

Im geladenen Zustand wird die zu verabreichende Produktdosis durch Verdrehen des Dosierelements 25 eingestellt, wobei grundsätzlich jede Produktdosis zwischen der Maximaldosis, wie sie aus der in Figur 1 dargestellten Einstellung verabreicht würde, und der Minimaldosis, wie sie im Falle der Einstellung der Figur 2 verabreicht würde, auswählbar ist. Allerdings rastet der Zeiger 31 bei seinem Entlanggleiten an der Mantelinnenfläche des Dosierelements 25 in mehreren diskreten Rastpositionen ein, die der Verwender durch ein Klickgeräusch wahrnimmt. Die Einstellung der Produktdosis in diskreten Schritten, hier mittels der mehreren auswählbaren Rastpositionen des Zeigers 31, erhöht die Sicherheit, dass auch tatsächlich die gewünschte Produktdosis eingestellt wird. Da das zweite Anzeigeelement 30 die axiale Einstellbewegung des Kolbenstangenteils 11 mitmacht, wird auf der Skala 26 unmittelbar die Position des Kolbenstangenteils 11 angezeigt.

Nach der Einstellung der Produktdosis oder auch vorher wird eine die Einstechkanüle 5 umhüllende Kanülenkappe abgenommen und aus der Gehäusehülse 2 herausgezogen. Der Injektor ist nun bereit für eine Injektion. Für die Injektion wird der Injektor im Bereich der Gehäusehülse 1 gegriffen und mit seinem distalen Ende an der Einstechstelle senkrecht auf die Haut aufgesetzt. Anschließend wird die Gehäusehülse 1 in die Vortriebsrichtung gedrückt, wodurch sie sich gegen die Kraft einer Feder 10 relativ zu der Gehäusehülse 2 in die Vortriebsrichtung V bewegt. Bei dieser Relativbewegung schiebt die einfahrende Gehäusehülse 2 das Übertragungsstück 22 in die axialen Überdeckung mit dem Auslöseelement 21 bis ein Axialanschlag erreicht ist. Der Axialanschlag begrenzt die Relativbewegung zwischen den Gehäusehülsen 1 und 2. Die Einstechkanüle 5 ragt nun bis fast an das distale Ende der Gehäusehülse 2.

Im nächsten Schritt wird die Antriebseinheit durch Drücken des Auslöseelements 21 ausgelöst. Das Auslöseelement 21 drückt radial gegen das Übertragungsstück 22, das in der erreichten Axialposition radial verschiebbar ist und seinerseits gegen das Blockierelement 20 rückt. Durch das Drücken des Auslöseelements 21 wird das Blockierelement 20 aus dem Halteeingriff mit der Vortriebsstruktur 16 gedrückt. Sobald der Halteeingriff gelöst ist, treibt die Einstechfeder 17 die Vortriebsstruktur 16 in die Vortriebsrichtung V. Die Vortriebsstruktur 16 ist in sich axial steif und drückt bei ihrer eigenen Vortriebsbewegung den Behältnishalter 6 mit dem darin aufgenommenen Behältnis 2 in die Vortriebsrichtung V. Die Einstechkanüle 5 wird durch diese Vortriebsbewegung in und vorzugsweise durch die Haut gestochen, um das Produkt zu verabreichen, vorzugsweise subkutan.

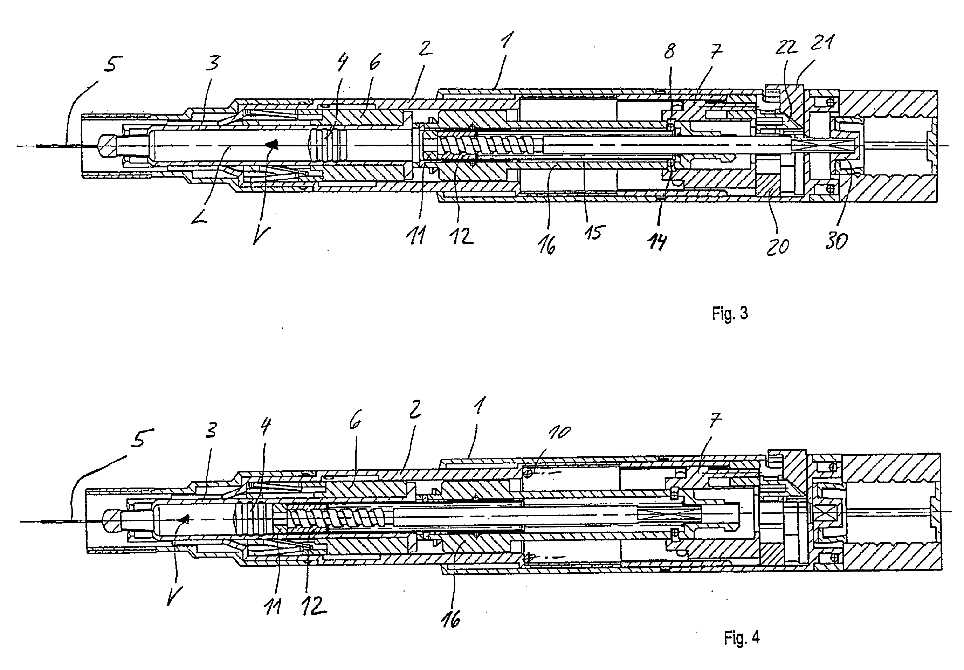

Figur 3 zeigt den Injektor in dem Zustand, in dem die Vortriebsbewegung des Behältnisses 2 und damit die Einstechbewegung der Einstechkanüle 5 gerade beendet ist, der Ausschütthub der Kolbenstange 11, 12 aber noch nicht eingesetzt hat. In diesem Zustand nach der Einstechphase und vor der Ausschüttphase sind die Durchbrechungen der Vortriebsstruktur 16, in denen die Blockierelemente 14 radial geführt werden, in die axiale Überdeckung mit Vertiefungen 8 oder einer einzigen umlaufenden Vertiefung 8 gelangt, die in der die Vortriebsstruktur 16 umgebenden Mantelinnenfläche des Lagerblocks 7 gebildet sind oder ist. Durch die nun gegebene Ausweichmöglichkeit und den geneigten Verlauf der Halteschulter 13 oder der mehreren Halteschultern 13 werden die Blockierelemente 14 entlang der Halteschulter 13 bzw. Halteschultern 13 nach außen gedrückt, so dass das Kolbenstangenteil 12 von den Blockierelementen 14 freikommt. Das Kolbenstangenteil 12 bewegt sich unter der Kraft der Ausschüttfeder 15 in die Vortriebsrichtung V und nimmt aufgrund des Dosiereingriffs das Kolbenstangenteil 11 mit. Nachdem die Kolbenstange 11, 12 die dem eingestellten Abstand zum Kolben 4 entsprechende Weglänge zurückgelegt und in Druckkontakt mit dem Kolben 4 gelangt ist, drückt sie den Kolben 4 im weiteren Verlauf des Ausschütthubs in dem Behältnis 2 in die Vortriebsrichtung V, wodurch die eingestellte Produktdosis über die Einstechkanüle 5 ausgeschüttet wird.

Bei dem Ausschütthub fährt auch das zweite Kolbenstangenteil 12 in das Behältnis 3 ein. Seine äußeren Abmaße im Querschnitt werden daher durch den Hohlquerschnitt des Behältnisses 3 begrenzt. Bei schlanken Behältnissen wird das zweite Kolbenstangenteil 12 daher vorzugsweise als Metallhülse 12b mit eingesetztem Eingriffsabschnitt 12a gebildet, da bei metallischen Hülsenkörpern mit geringerer Wandstärke die gleiche oder sogar eine größere Festigkeit als bei Kunststoffhülsen realisiert werden kann.

Im Verlauf des Ausschütthubs gelangt das Anzeigeelement 30 in Anschlag mit einer rückwärtigen Stirnfläche der Gehäusehülse 1, so dass sich die Kolbenstange 11, 12 von dem Anzeigeelement 30 löst.

Figur 4 zeigt den Injektor nachdem die eingestellte Produktdosis ausgeschüttet, aber bevor die Einstechkanüle 5 aus dem Gewebe herausgezogen wurde. Wird der Injektor von der Einstechstelle weggezogen, drückt die Feder 10 die Gehäusehülse 2 relativ zu der Gehäusehülse 1 in die Vortriebsrichtung V, bis sie in einer Anschlagposition die Einstechkanüle 5 über deren Spitze hinaus umgibt. In ihrer distalsten Position wird die Gehäusehülse 2 verriegelt, so dass Verletzungen durch die Einstechkanüle 5 ausgeschlossen werden. Der Injektor wird anschließend entsorgt, beispielsweise durch Zurückgabe an den Hersteller.

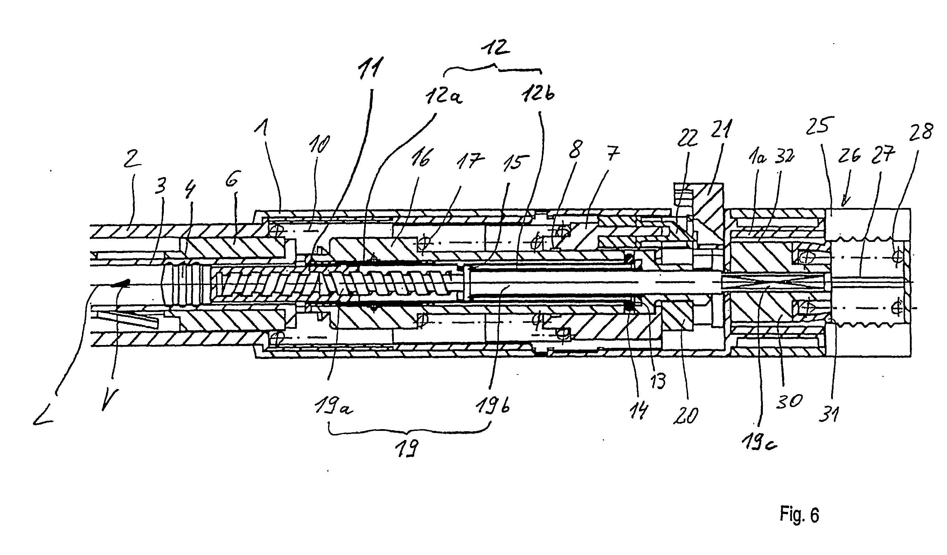

Figur 6 zeigt einen proximalen Teil eines dosisvariablen Autoinjektors nach einem zweiten Ausführungsbeispiel. Dargestellt ist der Injektor in der Einstellung "Maximaldosis". Für die ihrer Funktion nach jeweils gleichen Komponenten werden die gleichen Bezugszeichen verwendet wie im ersten Ausführungsbeispiel. Soweit nachfolgend nichts anderes beschrieben wird, sollen für das zweite Ausführungsbeispiel die Ausführungen zum ersten gelten.

Die Kolbenstange des zweiten Ausführungsbeispiels ist dreiteilig und besteht aus einem ersten Kolbenstangenteil 11, einem zweiten Kolbenstangenteil 12 und einem dritten Kolbenstangenteil 19. Das erste Kolbenstangenteil 11 bildet wieder den Stempel und einen Eingriffsabschnitt für den Dosiereingriff. Das erste Kolbenstangenteil 11 ist eine Hülse, die an ihrer Mantelinnenfläche den Eingriffsabschnitt aufweist und an ihrem proximalen Ende den Stempel als flachen Boden bildet. Der Eingriffsabschnitt ist wieder als Gewindeabschnitt gebildet. Der zweite Kolbenabschnitt 12 ist eine zylindrische Hülse und weist auch wieder einen Eingriffsabschnitt 12a und eine Haltehülse 12b mit der Halteschulter 13 an ihrem proximalen Ende auf. Das Kolbenstangenteil 12 umgibt das erste Kolbenstangenteil 11 und führt es mit seinem Eingriffsabschnitt 12a verdrehfrei axial linear. Das Kolbenstangenteil 12 ist seinerseits verdrehgesichert in der Vortriebsstruktur 16 aufgenommen, die wiederum verdrehgesichert von dem Lagerblock 7 geführt wird. Das dritte Kolbenstangenteil 19 besteht aus einem distalen Eingriffsabschnitt 19a, der mit dem ersten Kolbenstangenteil 11 in dem Dosiereingriff und im Ausführungsbeispiel ein Gewindeabschnitt ist, und einem proximalen Schaft 19b, den die Vortriebsstruktur 16 axial führt und dessen Ende als Kopplungsabschnitt 19c geformt ist. Das dritte Kolbenstangenteil 19 ist um die Längsachse L drehbar. Es wird von der Ausschüttfeder 15 in die Vortriebsrichtung V belastet, indem sich die Ausschüttfeder 15 an einer nach auswärts ragenden Schulter des dritten Kolbenstangenteils 19 abstützt. Die Schulter ist in Vortriebsrichtung auf Anschlag gegen eine Gegenschulter des zweiten Kolbenstangenteils 12, wodurch bei einem Auslösen des Injektors der Vortrieb der Kolbenstange 11, 12, 19 bewirkt wird. Somit stützt sich auch im zweiten Ausführungsbeispiel die Ausschüttfeder 15 letztlich an dem zweiten Kolbenstangenteil 12 ab.

Das dritte Kolbenstangenteil 19 ragt über eine rückwärtige Stirnfläche aus der Gehäusehülse 1 heraus und ist, wie auch bereits im ersten Ausführungsbeispiel, über das Anzeigeelement 30 und das Koppelelement 27 mit dem Dosierelement 25 verbunden. Das dritte Kolbenstangenteil 19 ist in der Halteposition der Kolbenstange 11, 12, 19 zwar drehbeweglich, ist aber zwischen der Ausschüttfeder 15 und der Anschlagschulter des zweiten Kolbenstangenteils 12 so fest eingespannt, dass für die praktischen Belange von einer axialen Fixierung in der Halteposition der Kolbenstange 11, 12, 19 gesprochen werden kann.

Das zweite Anzeigeelement 30 ist mit der Gehäusehülse 1 in einem Gewindeeingriff um die Längsachse L. Sein Gewindeabschnitt ist mit 32 bezeichnet, der Gewindeabschnitt der Gehäusehülse 1 mit 1a. Andererseits ist das Anzeigeelement 30 verdrehgesichert axial linear geführt mit dem Kolbenstangenteil 19 verbunden. Die Einstellung der Produktdosis wird wieder durch die Dosierdrehbewegung des Dosierelements 25 vorgenommen und mittels dessen Koppelelements 27 und des Anzeigeelements 30 auf das dritte Kolbenstangenteil 19 übertragen. Die Dosierbewegung wird in dem Dosiereingriff des Gewindeabschnitts 19a mit dem Gewindeabschnitt des ersten Kolbenstangenteils 11 und dessen axiale Linearführung in eine Axialbewegung des ersten Kolbenstangenteils 11 umgewandelt. Gleichzeitig wird das Anzeigeelement 30 in seinem Gewindeeingriff mit der Gehäusehülse 1 axial verstellt. Die Axialverstellung des Anzeigeelements 30 ist zu der Axialverstellung des ersten Kolbenstangenteils 11 proportional untersetzt, indem für den Gewindeeingriff des Anzeigeelements 30 eine geringere Gewindesteigung als für den Gewindeeingriff des ersten Kolbenstangenteils 11 gewählt ist. Das zweite Ausführungsbeispiel zeigt für die Dosisanzeige somit, wie das Maß der Axialverstellung der Kolbenstange 11, 12, 19 nahezu beliebig übersetzt oder vorzugsweise untersetzt in die Axialbewegung des Anzeigeelements 30 und damit des Zeigers 31 übertragen werden kann.

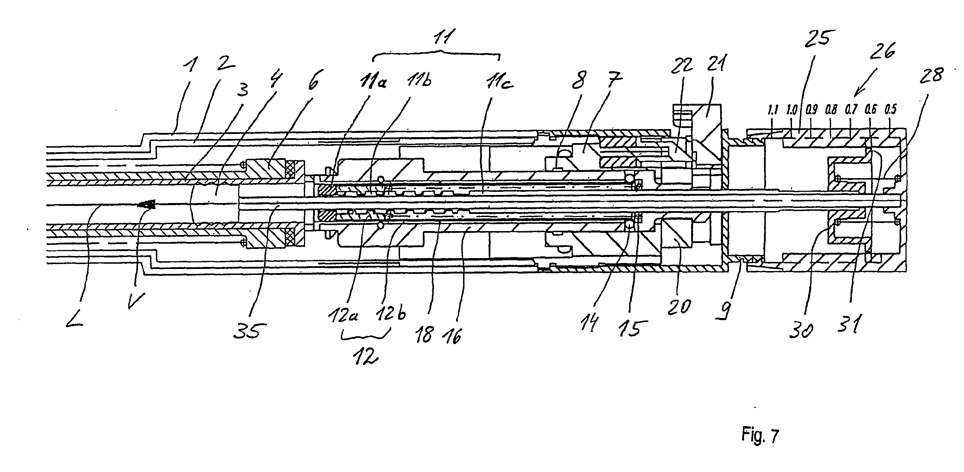

Figur 7 zeigt einen proximalen Teil eines dosisvariablen Autoinjektors nach einem dritten Ausführungsbeispiel. Der Autoinjektor ist in der Einstellung "Minimaldosis" dargestellt. Für die ihrer Funktion nach jeweils gleichen Komponenten werden die gleichen Bezugszeichen verwendet wie im ersten Ausführungsbeispiel. Die Antriebseinheit entspricht ihrer Funktion nach der Antriebseinheit des ersten Ausführungsbeispiels. Insbesondere ist die Kolbenstange 11, 12 wieder zweiteilig mit dem ersten Kolbenstangenteil 11 und dem zweiten Kolbenstangenteil 12 gebildet.

Im Unterschied zu den Injektoren der beiden ersten Ausführungsbeispiele ist der Autoinjektor des dritten Ausführungsbeispiels jedoch mit einer Primingeinrichtung 35 ausgestattet, mittels der die das Produkt führenden Komponenten bis zur Spitze der nicht dargestellten Einstechkanüle 5 vor der Injektion entlüftet werden können.

Die Primingeinrichtung 35 ist ein schlanker Stab und durchragt die Kolbenstange 11, 12 auf der Längsachse L. Beide Kolbenstangenteile 11 und 12 umgeben die Primingeinrichtung 35 konzentrisch. In der Halteposition der Kolbenstange 11, 12 durchragt die Primingeinrichtung 35 die Kolbenstange 11, 12 insbesondere in die Vortriebsrichtung V, so dass sie mit ihrem distalen Ende vor der Kolbenstange 11, 12 auf den Kolben 4 wirken kann. Sie durchragt die Kolbenstange 11, 12 aber auch gegen die Vortriebsrichtung V und ist an ihrem proximalen Ende mit dem Dosierelement 25 um die Längsachse L verdrehgesichert und axial unbeweglich verbunden. Die Kolbenstange 11, 12 unterscheidet sich von der Kolbenstange des ersten Ausführungsbeispiels nur dadurch, dass im dritten Ausführungsbeispiel das erste Kolbenstangenteil 11 hülsenförmig ist, um das Durchragen der Primingeinrichtung 35 zu ermöglichen. Die Primingeinrichtung 35 ist mit dem sie unmittelbar umgebenden ersten Kolbenstangenteil 11 um die Längsachse L verdrehgesichert verbunden, kann jedoch relativ zu dem ersten Kolbenstangenteil 11 und im Übrigen zu den weiteren Komponenten der Antriebseinheit in die Vortriebsrichtung V bewegt werden. Umgekehrt kann sich das Kolbenstangenteil 11 für die Einstellung der Produktdosis relativ zu der Primingeinrichtung 35 axial bewegen. Die Primingeinrichtung 35 kann relativ zu der Kolbenstange 11, 12 und natürlich auch relativ zu der Vortriebsstruktur 16 in deren jeweiligen Halteposition einen Priminghub ausführen, bei dem sie gegen den Kolben 4 drückend diesen in die Vortriebsrichtung V treibt. Die Länge des Priminghubs ist so bemessen, dass in den produktführenden Komponenten möglicherweise verbliebene Luft durch das Produkt sicher verdrängt wird, andererseits aber für diesen Verdrängungsvorgang möglichst wenig Produkt ausgeschüttet wird. Der Priminghub ist wesentlich kürzer als der Ausschütthub.

Das Dosierelement 25 dient nicht nur als Dosierelement für die Einstellung der Produktdosis, sondern auch als Betätigungselement für die Primingeinrichtung 35. Für die Betätigung der Primingeinrichtung 35 ist das Dosierelement 25 mit der Gehäusehülse 1 so verbunden, dass es relativ zu der Gehäusehülse 1 nicht nur die Dosierbewegung, sondern auch den Priminghub ausführen kann. Die Gehäusehülse 21 bildet für die Verbindung mit dem Dosierelement 25 an ihrem proximalen Ende einen Verbindungsabschnitt 9, der das Dosierelement 25 axial führt und mit dem das Dosierelement 25 in mehreren axial voneinander beabstandeten Rastpositionen verrasten kann. Der Verbindungsabschnitt 9 bildet einen Rastabschnitt mit Rastausnehmungen und das Dosierelement 25 eine entsprechende Rasteinrichtung, im Ausführungsbeispiel eine Rastnase 29. Im Auslieferungszustand ist das Dosierelement 25 in der proximalsten der Rastpositionen mit dem Verbindungsabschnitt 9 verrastet. Aus dieser Rastposition kann es in die Vortriebsrichtung V wahlweise in eine der anderen Rastpositionen bewegt werden. Ein hörbarer Klick zeigt dem Verwender das Verrasten an. Im Ausführungsbeispiel sind insgesamt 3 Rastpositionen vorgesehen. In jeder der Rastposition ist das Dosierelement 25 relativ zu der Gehäusehülse 1 drehbar.

Die Dosisanzeige berücksichtigt auch die axiale Position der Primingeinrichtung 35, d. h. sie berücksichtigt, ob ein Priminghub ausgeführt wurde und auch die Länge des ausgeführten Priminghubs. Die Dosisskala 26 ist hierfür um die Produktdosis erweitert, die durch einen Priminghub maximal ausschüttbar ist. Bei Ausführung eines Priminghubs bleibt das den Zeiger 31 bildende Anzeigeelement 30, das mit dem ersten Kolbenstangenteil 11 wie im ersten Ausführungsbeispiel verbunden ist, relativ zu der Gehäusehülse 1 axial stehen. Bei dem Priminghub wird allerdings die Dosisskala 26 relativ zu der Gehäusehülse 1 und dem Anzeigeelement 30 axial in die Vortriebsrichtung V bewegt, so dass sich die Position des Zeigers 31 auf der Dosisskala 26 entsprechend ändert. Wird das Dosierelement 26 aus seiner proximalsten Rastposition durch axialen Druck in seine distalste Rastposition bewegt, so dass die Primingeinrichtung 35 ihren größtmöglichen Priminghub ausführt, bewegt sich das Dosierelement 25 und damit dessen Dosisskala 26 relativ zu dem Anzeigeelement 30 und dessen Zeiger 31 in die Vortriebsrichtung V, und es wird eine nach Ausführung des Priminghubs noch verbleibende Restdosis angezeigt. Im Ausführungsbeispiel kann bei der Injektion in der dargestellten Einstellung "Minimaldosis" nach Ausführung des größtmöglichen Priminghubs noch die Hälfte des Behältnisinhalts ausgeschüttet werden. Im praktischen Gebrauch wird die Einstellung der Produktdosis nach der Betätigung der Primingeinrichtung 35, d. h. nach der Ausführung des Priminghubs, vorgenommen. Die Umkehrung der Reihenfolge ist jedoch ohne Weiteres möglich.

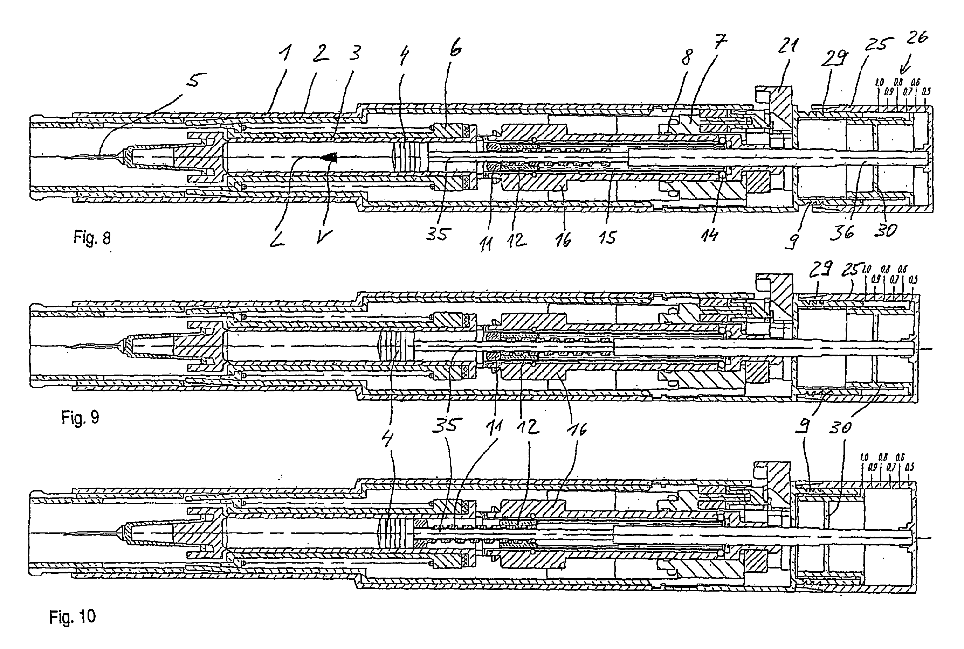

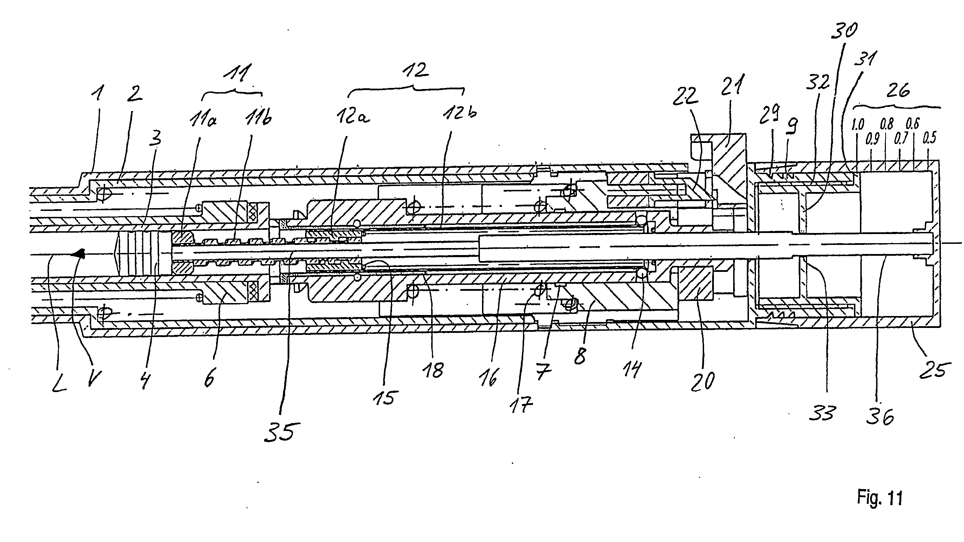

Die Figuren 8 bis 11 zeigen einen dosisvariablen Autoinjektor eines vierten Ausführungsbeispiels, der ebenfalls mit einer Primingeinrichtung 35 ausgestattet ist. Das vierte Ausführungsbeispiel unterscheidet sich hinsichtlich der Primingeinrichtung 35 vom dritten Ausführungsbeispiel dadurch, dass mit der Primingeinrichtung 35 eine Dosisanzeige wie bei dem zweiten Ausführungsbeispiel gebildet wird. Die Figuren 8 bis 10 zeigen den Injektor in unterschiedlichen axialen Positionen der Primingeinrichtung 35 und den beiden extremen Einstellungen der Kolbenstange 11, 12. Figur 11 zeigt den proximalen Teil des Injektors in der Einstellung der Figur 10 in einer Vergrößerung. Begleitend zu den Figuren 8 bis 10 sei stets auch auf Figur 11 verwiesen.

Die Kolbenstange 11, 12 ist wieder zweiteilig, wobei das zweite Kolbenstangenteil 12 wie im ersten und im dritten Ausführungsbeispiel gebildet ist. Das erste Kolbenstangenteil 11 besteht aus dem Stempel 11a und dem Eingriffsabschnitt 11b, die wie im dritten Ausführungsbeispiel geformt sind. Es weist lediglich die axiale Länge auf, die erforderlich ist, um es in dem Dosiereingriff mit dem zweiten Kolbenstangenteil 12 in dessen Halteposition aus der in Figur 8 dargestellten Einstellung für die Minimaldosis in die in Figur 10 dargestellte Einstellung für die Maximaldosis, in der es den Kolben 4 gerade kontaktiert, verstellen zu können. Das zweite Kolbenstangenteil 12 bildet je wieder einen Anschlag, der die axiale Verstellung des ersten Kolbenstangenteils 11 in und gegen die Vortriebsrichtung V begrenzt. Das erste Kolbenstangenteil 11 ist in seinem Eingriffsabschnitt 11b eine dünne Hülse und ist wie im dritten Ausführungsbeispiel mit der Primingeinrichtung 35 verdrehgesichert, aber axial bewegbar verbunden. Die Primingeinrichtung 35 ist ihrerseits mit dem Dosierelement 25 verdrehgesichert verbunden und bildet so wieder die Kopplung zwischen dem Dosierelement 25 und der Kolbenstange 11, 12.

Im Unterschied zum dritten Ausführungsbeispiel ist das Anzeigeelement 30 relativ zu der Primingeinrichtung 35 axial bewegbar und wird von der Primingeinrichtung 35 verdrehgesichert axial geführt. Der Führungsabschnitt der Primingeinrichtung 35 ist mit 36 bezeichnet und der des Anzeigeelements 30 mit 33. Mit der Gehäusehülse 1 besteht ein Gewindeeingriff bei 1a und 32. Die Dosisanzeige des vierten Ausführungsbeispiels entspricht somit derjenigen des zweiten Ausführungsbeispiels (Figur 6), wobei die Primingeinrichtung 35 diesbezüglich die Funktion des dritten Kolbenstangenteils 19 des zweiten Ausführungsbeispiels ausübt.

Figur 8 zeigt den Injektor in einem Zustand, in dem die bei der Injektion ausschüttbare Minimaldosis eingestellt und der Injektor noch nicht entlüftet ist. Zum Entlüften drückt der Verwender das Dosierelement 25 aus seiner dargestellten proximalsten Rastposition mit dem Verbindungsabschnitt 9 in Vortriebsrichtung V in eine der anderen Rastpositionen. Die Primingeinrichtung 35 wird durch diese Betätigung um ein durch die Rastpositionen vorgegebenes Wegstück in die Vortriebsrichtung V bewegt und stößt dabei auch den Kolben 4 in die Vortriebsrichtung V, so dass etwaige Restluft im System durch das Produkt verdrängt wird.

Figur 9 zeigt den Injektor mit eingestellter Minimaldosis und nach Ausführung eines größtmöglichen Priminghubs. Die Dosisanzeige zeigt in diesem Zustand an, dass bei der Injektion die Hälfte der maximal ausschüttbaren Produktdosis ausgeschüttet wird.

Figur 10 zeigt den Injektor in der Einstellung "Maximaldosis" und nach Ausführung eines größtmöglichen Priminghubs. Die Dosisanzeige zeigt an, dass im Falle der Auslösung des Injektors in diesem Zustand die maximal ausschüttbare Produktdosis ausgeschüttet wird.

Die Injektion wird bei sämtlichen Ausführungsbeispielen durchgeführt wie anhand des ersten Ausführungsbeispiels beschrieben.

### Bezugszeichen

- 1: Gehäusehülse
- 1a: Gewindeabschnitt
- 2: Gehäusehülse, Nadelschutz
- 3: Behältnis
- 4: Kolben
- 5: Einstechkanüle
- 6: Behältnishalter
- 7: Lagerblock
- 8: Vertiefung
- 9: Verbindungsabschnitt, Rastabschnitt
- 10: Feder
- 11: erstes Kolbenstangenteil
- 11a: Stempel
- 11b: Eingriffsteil, Gewindeabschnitt
- 11c: Schaft
- 11d: Kopplungsabschnitt
- 12: zweites Kolbenstangenteil
- 12a: Eingriffsteil, Gewindemutter
- 12b: Haltehülse
- 13: Halteschulter
- 14: Blockierelement, Zylinderstift
- 15: Ausschüttfeder
- 16: Vortriebsstruktur
- 17: Einstechfeder
- 18: Ausschüttanschlag
- 19: drittes Kolbenstangenteil
- 19a: Eingriffsabschnitt
- 19b: Schaft
- 19c: Kopplungsabschnitt
- 20: Blockierelement
- 21: Auslöseelement, Knopf
- 22: Übertragungsstück
- 23: -
- 24: -
- 25: Dosierelement, Anzeigeelement
- 26: Dosisskala
- 27: Koppelelement
- 28: Feder
- 29: Rasteinrichtung, Rastnase
- 30: Anzeigeelement
- 31: Zeiger
- 32: Eingriffsabschnitt, Gewindeabschnitt
- 33: Führungsabschnitt
- 34: -
- 35: Primingeinrichtung, Primingstab
- 36: Führungsabschnitt

## Patentansprüche

1. Vorrichtung für die Verabreichung eines injizierbaren Produkts, umfassend:
a) ein Gehäuse (1),
b) ein Reservoir (3) für das Produkt,
c) einen in dem Reservoir (3) mittels einer Kolbenstange (11, 12) der Vorrichtung in eine Vortriebsrichtung (V) axial bewegbaren Kolben, der für eine Produktausschüttung einen Ausschütthub in die Vortriebsrichtung (V) ausführt,
d) und eine Primingeinrichtung (35), die auf den Kolben (4) wirkend in die Vortriebsrichtung (V) bewegbar ist, um für ein Entlüften des Reservoirs (3) einen Priminghub in die Vortriebsrichtung (V) ausführen zu können, wobei
e) die Primingeinrichtung (35) für den Priminghub in die Vortriebsrichtung (V) relativ zu der Kolbenstange (11, 12) bewegbar ist.
**dadurch gekennzeichnet, dass** die Primingeinrichtung (35) die Kolbenstange (11, 12) in die Vortriebsrichtung (V) durchragt.

2. Vorrichtung nach Anspruch 1, **dadurch gekennzeichnet, dass** die Primingeinrichtung (35) unabhängig von der Kolbenstange (11, 12) betätigbar ist.

3. Vorrichtung nach einem der vorhergehenden Ansprüche, **dadurch gekennzeichnet, dass** die Primingeinrichtung (35) in die Vortriebsrichtung (V) linear geführt wird.

4. Vorrichtung nach einem der vorhergehenden Ansprüche, **dadurch gekennzeichnet, dass** die Vorrichtung ein Dosierelement (25) umfasst, das an dem Gehäuse (1) bewegbar angeordnet und über einen Dosiereingriff mit der Kolbenstange (11, 12) so gekoppelt ist, dass eine Dosierbewegung des Dosierelements (25) eine Veränderung eines axialen Abstands, den die Kolbenstange (11, 12) zu dem Kolben (4) aufweist, oder eine Veränderung der axialen Länge des Ausschütthubs bewirkt.

5. Vorrichtung nach dem vorhergehenden Anspruch, **dadurch gekennzeichnet, dass** das Dosierelement (25) ein Betätigungselement für die Primingeinrichtung (35) bildet.

6. Vorrichtung nach dem vorhergehenden Anspruch, **dadurch gekennzeichnet, dass** das Dosierelement (25) in die Vortriebsrichtung (V) bewegbar angeordnet ist und bei einer Bewegung in die Vortriebsrichtung (V) die Primingeinrichtung (35) mitnimmt.

7. Vorrichtung nach einem der drei vorhergehenden Ansprüche, **dadurch gekennzeichnet, dass** die Kolbenstange (11, 12) ein auf den Kolben (4) wirkendes erstes Kolbenstangenteil (11) und ein zweites Kolbenstangenteil (12) umfasst und dass eines der Kolbenstangenteile (11, 12) relativ zu dem anderen axial verstellbar ist.

8. Vorrichtung nach dem vorhergehenden Anspruch, **dadurch gekennzeichnet, dass** die Kolbenstangenteile (11, 12) miteinander in dem Dosiereingriff sind.

9. Vorrichtung nach einem der fünf vorhergehenden Ansprüche, **dadurch gekennzeichnet, dass** die Kolbenstange (11, 12) die Dosierbewegung des Dosierelements (25) mitmacht.

10. Vorrichtung nach einem der sechs vorhergehenden Ansprüche, **dadurch gekennzeichnet, dass** die Primingeinrichtung (35) das Dosierelement (25) mit der Kolbenstange (11, 12) koppelt, indem sie die Dosierbewegung auf die Kolbenstange (11, 12) überträgt.

11. Vorrichtung nach einem der sieben vorhergehenden Ansprüche, **dadurch gekennzeichnet, dass** der Dosiereingriff ein Gewindeeingriff um eine in die Vortriebsrichtung (V) weisende Achse (L) ist.

12. Vorrichtung nach einem der vorhergehenden Ansprüche, **dadurch gekennzeichnet, dass** die Kolbenstange (11, 12) mit dem Kolben (4) nur Druckkontakt hat.

13. Vorrichtung nach einem der vorhergehenden Ansprüche, **dadurch gekennzeichnet, dass** die Primingeinrichtung (35) mit dem Kolben (4) nur Druckkontakt hat.

14. Vorrichtung nach einem der vorhergehenden Ansprüche, **dadurch gekennzeichnet, dass** die Vorrichtung eine Ausschüttfeder (15) umfasst, die in die Vortriebsrichtung (V) auf die Kolbenstange (11, 12) wirkt und dass die Kolbenstange (11, 12) in einer Halteposition gegen die Kraft der Ausschüttfeder (15) in einem lösbaren Halteeingriff blockiert ist.

15. Vorrichtung nach dem vorhergehenden Anspruch in Kombination mit einem der Ansprüche 9 und 10, **dadurch gekennzeichnet, dass** das zweite Kolbenstangenteil (12) in dem Halteeingriff blockiert ist.

16. Vorrichtung nach einem der zwei vorhergehenden Ansprüche, **dadurch gekennzeichnet, dass** die Vorrichtung ein Autoinjektor ist.

17. Vorrichtung nach dem vorhergehenden Anspruch, ferner umfassend
eine in die Vortriebsrichtung (V) weisende Einstechkanüle (5), die mit dem in die Vortriebsrichtung (V) bewegbaren Reservoir (3) verbunden ist,
und eine axial bewegbare Struktur (16), die das Reservoir in die Vortriebsrichtung bewegbar lagert oder die vorzugsweise in einer Halteposition gegen die Kraft einer Einstechfeder (17) in einem lösbaren Halteeingriff blockiert ist und bei einem Lösen des Halteeingriffs das Reservoir (3) und die Einstechkanüle (5) in die Vortriebsrichtung (V) treibt.

18. Vorrichtung nach einem der vorhergehenden Ansprüche, **dadurch gekennzeichnet, dass** die Vorrichtung eine Dosisanzeige mit wenigstens zwei Anzeigeelementen (25, 30) umfasst, von denen das eine (25) eine Dosisskala (26) und das andere (30) einen Zeiger (31) der Dosisanzeige bildet, und dass eines der Anzeigeelemente (25, 30) mit der Primingeinrichtung (35) so gekoppelt ist, dass die Position des Zeigers (31) bei einem Priminghub auf der Dosisskala (36) verstellt wird.

19. Vorrichtung nach dem vorhergehenden Anspruch, **dadurch gekennzeichnet, dass** eines der Anzeigeelemente (25, 30) ein Betätigungselement für die Primingeinrichtung (35) bildet.

20. Vorrichtung nach dem vorhergehenden Anspruch, **dadurch gekennzeichnet, dass** das Anzeigeelement (25), das das Betätigungselement bildet, in einem durch den Priminghub lösbaren Rasteingriff (9, 29) mit dem Gehäuse (1) ist.

21. Vorrichtung nach einem der drei vorhergehenden Ansprüche, **dadurch gekennzeichnet, dass** wenigstens eines der Anzeigeelemente (25, 30) mit der Kolbenstange (11, 12) so gekoppelt ist, dass eine axiale Verstellung der Kolbenstange (11, 12) eine Verstellung des gekoppelten Anzeigeelements (30) relativ zu dem anderen Anzeigeelement (25) bewirkt.

22. Vorrichtung nach dem vorhergehenden Anspruch, **dadurch gekennzeichnet, dass** das mit der Kolbenstange (11, 12) gekoppelte Anzeigeelement (30) die axiale Verstellung der Kolbenstange (11, 12) mitmacht.

23. Vorrichtung nach Anspruch 23, **dadurch gekennzeichnet, dass** die Kolbenstange (11, 12) mit dem wenigstens einen der Anzeigeelemente (25, 30) mittels eines Getriebes (32, 36) gekoppelt ist.

24. Vorriclitung nach dem vorhergehenden Anspruch, **dadurch gekennzeichnet, dass** die Primingeinrichtung (35) das wenigstens eine (25) der Anzeigeelemente (25, 30) mit der Kolbenstange (11, 12) koppelt.

25. Vorrichtung nach einem der sieben vorhergehenden Ansprüche, **dadurch gekennzeichnet, dass** eines der Anzeigeelemente (25, 30) das andere umgibt und zumindest in einem Bereich, der die Dosisskala (26) bildet oder umgibt, durchsichtig ist.

26. Vorrichtung nach einem der acht vorhergehenden Ansprüche in Kombination mit Anspruch 6, **dadurch gekennzeichnet, dass** das Dosierelement (25) eines der Anzeigeelemente (25, 30) bildet.

## Claims

1. A device for administering an injectable product, comprising:
a) a housing (1);
b) a reservoir (3) for the product;
c) a piston which can be axially moved in the reservoir (3) by means of a piston rod (11, 12) of the device and which performs a delivery stroke in the advancing direction (V) in order to deliver product;
d) and a priming means (35) which can be moved in the advancing direction (V), acting on the piston (4), in order to be able to perform a priming stroke in the advancing direction (V) in order to vent the reservoir (3), wherein
e) the priming means (35) can be moved relative to the piston rod (11, 12) for the priming stroke in the advancing direction (V),
**characterised in that**
f) the priming means (35) protrudes through the piston rod (11, 12) in the advancing direction (V).

2. The device according to claim 1, **characterised in that** the priming means (35) can be actuated independently of the piston rod (11, 12).

3. The device according to any one of the preceding claims, **characterised in that** the priming means (35) is linearly guided in the advancing direction (V).

4. The device according to any one of the preceding claims, **characterised in that** the device comprises a dosing element (25) which is movably arranged on the housing (1) and coupled to the piston rod (11, 12) via a dosing engagement, such that a dosing movement of the dosing element (25) causes a change in an axial distance which the piston rod (11, 12) exhibits from the piston (4) or a change in the axial length of the delivery stroke.

5. The device according to the preceding claim, **characterised in that** the dosing element (25) forms an actuating element for the priming means (35).

6. The device according to the preceding claim, **characterised in that** the dosing element (25) is arranged such that it can be moved in the advancing direction (V), and slaves the priming means (35) when moving in the advancing direction (V).

7. The device according to any one of the preceding three claims, **characterised in that** the piston rod (11, 12) comprises a first piston rod portion (11), which acts on the piston (4), and a second piston rod portion (12), and **in that** one of the piston rod portions (11, 12) can be axially adjusted relative to the other.

8. The device according to the preceding claim, **characterised in that** the piston rod portions (11, 12) are in dosing engagement with each other.

9. The device according to any one of the preceding five claims, **characterised in that** the piston rod (11, 12) is slaved in the dosing movement of the dosing element (25).

10. The device according to any one of the preceding six claims, **characterised in that** the priming means (35) couples the dosing element (25) to the piston rod (11, 12) by transferring the dosing movement onto the piston rod (11, 12).

11. The device according to any one of the preceding seven claims, **characterised in that** the the dosing engagement is a threaded engagement about an axis (L) which points in the advancing direction (V).

12. The device according to any one of the preceding claims, **characterised in that** the piston rod (11, 12) has only pressing contact with the piston (4).

13. The device according to any one of the preceding claims, **characterised in that** the priming means (35) has only pressing contact with the piston (4).

14. The device according to any one of the preceding claims, **characterised in that** the device comprises a delivery spring (15) which acts on the piston rod (11, 12) in the advancing direction (V), and **in that** in a holding position, the piston rod (11, 12) is blocked in a releasable holding engagement, against the force of the delivery spring (15).

15. The device according to the preceding claim in combination with any one of claims 9 and 10, **characterised in that** the second piston rod portion (12) is blocked in the holding engagement.

16. The device according to any one of the preceding two claims, **characterised in that** the device is an auto-injector.

17. The device according to the preceding claim, further comprising:
an injection cannula (5) which points in the advancing direction (V) and is connected to the reservoir (3) which can be moved in the advancing direction (V);
and a structure (16) which can be axially moved and mounts the reservoir such that it can be moved in the advancing direction (V) or, in a holding position, is preferably blocked in a releasable holding engagement against the force of an injection spring (17) and, when the holding engagement is released, drives the reservoir (3) and the injection cannula (5) in the advancing direction (V).

18. The device according to any one of the preceding claims, **characterised in that** the device comprises a dosage display comprising at least two display elements (25, 30), one (25) of which forms a dosage scale (26) and the other (30) of which forms an indicator (31) of the dosage display, and **in that** one of the display elements (25, 30) is coupled to the priming means (35) such that the position of the indicator (31) is adjusted on the dosage scale (26) during a priming stroke.

19. The device according to the preceding claim, **characterised in that** one of the display elements (25, 30) forms an actuating element for the priming means (35).

20. The device according to the preceding claim, **characterised in that** the display element (25) which forms the actuating element is in a locking engagement (9, 29) with the housing (1) which can be released by the priming stroke.

21. The device according to any one of the preceding three claims, **characterised in that** at least one of the display elements (25, 30) is coupled to the piston rod (11, 12) such that an axial adjustment of the piston rod (11, 12) causes an adjustment of the coupled display element (30) relative to the other display element (25).

22. The device according to the preceding claim, **characterised in that** the display element (30) which is coupled to the piston rod (11, 12) is slaved in the axial adjustment of the piston rod (11, 12).

23. The device according to claim 23, **characterised in that** the piston rod (11, 12) is coupled to the at least one of the display elements (25, 30) by means of a gear (32, 36).

24. The device according to the preceding claim, **characterised in that** the priming means (35) couples the at least one (25) of the display elements (25, 30) to the piston rod (11, 12).

25. The device according to any one of the preceding seven claims, **characterised in that** one of the display elements (25, 30) surrounds the other and is transparent, at least in a region which forms or surrounds the dosage scale (26).

26. The device according to any one of the preceding eight claims in combination with claim 6, **characterised in that** the dosing element (25) forms one of the display elements (25, 30).

## Revendications

1. Dispositif pour administrer un produit à injecter, comprenant :
a) un boîtier (1),
b) un réservoir (3) pour le produit,
c) un piston pouvant être déplacé de manière axiale dans le réservoir (3) au moyen d'une tige de piston (11, 12) du dispositif dans un sens de poussée vers l'avant (V), lequel piston exécute une course d'administration dans le sens de poussée vers l'avant (V) pour l'administration du produit,
d) et un dispositif d'amorçage (35) qui peut être déplacé sur le piston (4) de manière active dans le sens de la poussée vers l'avant (V), afin de pouvoir exécuter une course d'amorçage pour une purge du réservoir (3) dans le sens de la poussée vers l'avant (V), moyennant quoi
e) le dispositif d'amorçage (35) peut être déplacé pour la course d'amorçage dans le sens de la poussée vers l'avant (V) par rapport à la tige de piston (11, 12), **caractérisé en ce que** le dispositif d'amorçage (35) fait saillie dans la tige du piston (11, 12) dans le sens de la poussée vers l'avant (V).

2. Dispositif selon la revendication 1, **caractérisé en ce que** le dispositif d'amorçage (35) peut être actionné indépendamment de la tige du piston (11, 12).

3. Dispositif selon l'une des revendications précédentes, **caractérisé en ce que** le dispositif d'amorçage (35) est entraîné de façon linéaire dans le sens de la poussée vers l'avant (V)

4. Dispositif selon l'une des revendications précédentes, **caractérisé en ce que** le dispositif comprend un élément de dosage (25) qui est disposé de manière mobile sur le boîtier (1) et qui est couplé avec la tige du piston (11, 12) via une prise de dosage de sorte qu'un mouvement de dosage de l'élément de dosage (25) entraîne une modification d'un écart axial entre la tige du piston (11, 12) et le piston (4) ou une modification de la longueur axiale de la course d'administration.

5. Dispositif selon l'une des revendications précédentes, **caractérisé en ce que** l'élément de dosage (25) forme un élément d'actionnement pour le dispositif d'amorçage (35).

6. Dispositif selon l'une des revendications précédentes, **caractérisé en ce que** l'élément de dosage (25) est disposé de manière mobile dans le sens de la poussée vers l'avant (V) et lors d'un mouvement entraîne le dispositif d'amorçage (35) dans le sens de la poussée vers l'avant (V).

7. Dispositif selon l'une des trois revendications précédentes, **caractérisé en ce que** la tige du piston (11, 12) comprend une première pièce de la tige de piston agissant sur le piston (4) et une deuxième pièce de la tige de piston (12), et **en ce qu'**une des pièces de la tige de piston (11, 12) peut-être réglée par rapport à l'autre de manière axiale.

8. Dispositif selon l'une des revendications précédentes, **caractérisé en ce que** les pièces de la tige du piston (11, 12) sont l'une avec l'autre dans la prise de dosage.

9. Dispositif selon l'une des cinq revendications précédentes, **caractérisé en ce que** la tige du piston (11, 12) participe au mouvement de dosage de l'élément de dosage (25).

10. Dispositif selon l'une des six revendications précédentes, **caractérisé en ce que** le dispositif d'amorçage (35) couple l'élément de dosage (25) avec la tige du piston (11, 12) en communiquant le mouvement de dosage à la tige du piston (11, 12).

11. Dispositif selon l'une des sept revendications précédentes, **caractérisé en ce que** la prise de dosage est une prise de filetage autour d'un axe (L) indiquant le sens de la poussée vers l'avant (V).

12. Dispositif selon l'une des revendications précédentes, **caractérisé en ce que** la tige du piston (11, 12) a uniquement un contact à pression avec le piston (4).

13. Dispositif selon l'une des revendications précédentes, **caractérisé en ce que** le dispositif d'amorçage (35) a uniquement un contact à pression avec le piston (4).

14. Dispositif selon l'une des revendications précédentes, **caractérisé en ce que** le dispositif comprend un ressort d'administration (15) qui agit sur la tige du piston (11, 12) dans le sens de la poussée vers l'avant (V) et **en ce que** la tige du piston (11, 12) est bloquée en position de retenue contre la force du ressort d'administration (15) dans une prise de retenue détachable.

15. Dispositif selon la revendication précédente en combinaison avec l'une des revendications 9 et 10, **caractérisé en ce que** la deuxième partie de la tige du piston (12) est bloquée en prise de retenue.

16. Dispositif selon l'une des deux revendications précédentes, **caractérisé en ce que** le dispositif est un auto-injecteur.

17. Dispositif selon la revendication précédente, comprenant en outre une canule d'insertion (5) indiquant le sens de la poussée vers l'avant (V) qui est reliée au réservoir (3) mobile dans le sens de la poussée vers l'avant (V), et une structure (16) mobile de manière axiale qui loge le réservoir de façon mobile dans le sens de la poussée vers l'avant ou qui est bloquée de préférence dans une position de retenue contre la force d'un ressort d'insertion (17) dans une prise de retenue amovible et qui, en cas de relâchement de la prise de retenue entraîne le réservoir (3) et la canule d'insertion (5) dans le sens de la poussée vers l'avant (V).

18. Dispositif selon l'une des revendications précédentes, **caractérisé en ce que** le dispositif comprend un écran de dosage avec au moins deux éléments d'affichage (25, 30), dont l'un (25) constitue une échelle de dosage (26) et l'autre (30) une aiguille (31) de l'écran de dosage, et **en ce qu'**un des éléments d'affichage (25, 30) est couplé avec le dispositif d'amorçage (35) de sorte que la position de l'aiguille est réglée sur l'échelle de dosage (36) en cas de course d'amorçage.

19. Dispositif selon la revendication précédente, **caractérisé en ce qu'**un des éléments d'affichage (25, 30) constitue un élément d'actionnement pour le dispositif d'amorçage (35).

20. Dispositif selon l'une des revendications précédentes, **caractérisé en ce que** l'élément d'affichage (25), qui constitue l'élément d'actionnement, est dans une prise d'enclenchement (9, 29) avec le boîtier pouvant être détachée par la course d'amorçage.

21. Dispositif selon l'une des trois revendications précédentes, **caractérisé en ce qu'**au moins un des éléments d'affichage (25, 30) est couplé avec la tige du piston (11, 12), de sorte qu'un réglage axial de la tige du piston (11, 12) induit un réglage de l'élément d'affichage couplé (30) par rapport à l'autre élément d'affichage (25).

22. Dispositif selon la revendication précédente, **caractérisé en ce que** l'élément d'affichage (30) couplé à la tige du piston (11, 12) participe au réglage axial de la tige du piston (11, 12).

23. Dispositif selon la revendication 23, **caractérisé en ce que** la tige du piston (11, 12) est couplée avec le au moins un des éléments d'affichage (25, 30) au moyen d'un organe de commande (32, 36).

24. Dispositif selon la revendication précédente, **caractérisé en ce que** le dispositif d'amorçage (35) couple le au moins un (25) des éléments d'affichage (25, 30) à la tige du piston (11, 12).

25. Dispositif selon l'une des sept revendications précédentes, **caractérisé en ce que** l'un des éléments d'affichage (25, 30) entoure l'autre et est transparent au moins dans une zone qui forme ou entoure l'échelle de dosage (26).

26. Dispositif selon l'une des huit revendications précédentes en combinaison avec la revendication 6, **caractérisé en ce que** l'élément de dosage (25) constitue un des éléments d'affichage (25, 30).
